# EUROPEAN PATENT APPLICATION

(11) **EP 3 904 841 A1**
(43) Date of publication of application: **03.11.2021**
(21) Application number: 19904345.6
(22) Date of filing: 14.11.2019
(51) Int. Cl.: G01F 11/28, G01N 1/10, G01N 1/38

(54) **FLUID MEASUREMENT DEVICE AND SAMPLE ANALYSIS DEVICE COMPRISING SAME**

(30) Priority: 27.12.2018 JP 2018246184
(71) Applicant: HORIBA, Ltd., Kyoto 601-8510 (JP)
(72) Inventor: SAKATO, Akiko, Kyoto-shi, Kyoto 601-8510 (JP); OKU, Narihiro, Kyoto-shi, Kyoto 601-8510 (JP)
(74) Representative: Isarpatent
(86) International application number: PCT/JP2019/044692
(87) International publication number: WO 2020/137226

(57) **Abstract**

First and second valves are connected to first and second parts of a volumetric measurement conduit with a predetermined volume. Each valve has first and second ports, an internal volume of the first port is smaller than an internal volume of the second port when the valves are closed, and the first port is connected to an end part of the volumetric measurement conduit, whereby a fluid volumetric measurement device is constituted. As a result, the amount of a sample liquid that enters the port of the valve can be reduced, and the accuracy of volumetric measurement is improved. When the fluid volumetric measurement device is used as a dilution mechanism, a sample analysis device using an inexpensive valve, requiring a short operating time, and suppressing a decrease in the dilution accuracy is provided.

## Description

### [Technical Field]

The present invention relates to a fluid volumetric measurement device for volumetrically measuring a fluid with a predetermined volume, and a sample analysis device having the fluid volumetric measurement device.

### [Background Art]

As a device for diluting sample liquids, a dilution device having a given length of a piping tube and an electromagnetic valve provided on both ends of the piping tube is known (patent document 1). The dilution device has a volumetric measurement tube (tube) with a given length and a given inner diameter, and has an electromagnetic valve connected to the both ends of the volumetric measurement tube. By opening and closing of the electromagnetic valves at both ends and operation of the external pump, a predetermined amount of sample liquid is filled in the volumetric measurement tube, and extruded by a predetermined amount of a diluent. The predetermined amount of the sample liquid is sent to a container together with the predetermined amount of the diluent, and becomes a sample liquid diluted to a predetermined concentration. The dilution device as described in patent document 1 is used when the volume of the sample liquid to be diluted is relatively large, as is clear from the fact that the volumetric measurement tube is wound in a loop.

On the other hand, in blood analysis device and the like, the amount of specimen liquid to be diluted is smaller, and dilution with higher accuracy is required (e.g., patent document 2 and the like). Therefore, it is important to volumetrically measure liquids with higher accuracy.

### [Document List]

### [Patent documents]

patent document 1: JP 2012-220246 A
patent document 2: JP 2007-93308 A
patent document 3: US 2008/0006336 A1

### [Summary of Invention]

### [Technical Problem]

For example, in conventional blood analysis devices, blood specimens are diluted more thinly in erythrocyte analysis than in leukocyte analysis due to the difference in the numbers of leukocytes and erythrocytes in the blood. To be specific, a sampling nozzle first sucks a blood specimen from a specimen container, and discharges a predetermined amount of the blood specimen into a chamber (WBC chamber) for measuring leukocytes. A diluent supply device adds a predetermined amount of diluent thereto (primary dilution), then a sampling nozzle sucks a predetermined amount of the primarily-diluted blood specimen (sample liquid) in the WBC chamber, and discharges same into a chamber (RBC chamber) for measuring erythrocytes. Then, the diluent supply device adds a predetermined amount of diluent thereto (secondary dilution).

The present inventors took note of the fact that the aforementioned step for the secondary dilution includes a dispensing action of the sampling nozzle (descending into WBC chamber, suction of sample liquid, ascending, moving, descending into RBC chamber, discharge of sample liquid) and a series of these actions takes a comparatively long time, and considered to apply a dilution device as described in patent document 1 for the secondary dilution of the sample liquid with the aim of further shortening the time of the action.

However, when the dilution device described in patent document 1 was actually applied, it was newly found that the accuracy of the concentration of the secondarily-diluted sample liquid sometimes becomes low. The present inventors investigated in detail the cause of the decrease in the accuracy of the concentration after dilution, and found that the volume of the volumetrically measured sample liquid varies due to the mechanism of volumetric measurement. That is, in the conventional mechanism for volumetric measurement, the sample liquid remaining in a part from the both ends of the volumetric measurement tube to the inside of a port of the electromagnetic valve connected to each end is mixed with the diluent when the sample liquid in the volumetric measurement tube is fed out by the diluent, which causes an error in the amount of the liquid to be volumetrically measured, thus also lowering the accuracy of the dilution rate.

The aforementioned problems of the volumetric measurement accuracy similarly occur not only in blood analysis devices but also devices requiring volumetric measurement of fluid and the like.

The problem of the present invention is to solve the aforementioned problems, provide a fluid volumetric measurement device that suppresses a decrease in the volumetric measurement accuracy of fluid, and provide a sample analysis device provided with the fluid volumetric measurement device.

### [Solution to Problem]

The main constitution of the present invention is as follows.
[1] A fluid volumetric measurement device comprising at least a volumetric measurement conduit with a predetermined volume, a first valve connected to a first part of the volumetric measurement conduit, and a second valve connected to a second part of the volumetric measurement conduit, wherein
   the first valve and the second valve each have a first port and a second port, an internal volume of the first port is smaller than an internal volume of the second port when the valves are closed,
   the first port of the first valve is connected to the first part of the volumetric measurement conduit, and the first port of the second valve is connected to the second part of the volumetric measurement conduit.
[2] The fluid volumetric measurement device of the aforementioned [1], wherein the first valve is a valve for openably/closably connecting the first part of the volumetric measurement conduit to a first conduit communicating with a first container containing a first fluid,
   the second valve is a valve for openably/closably connecting the second part of the volumetric measurement conduit to a second conduit to be an exit of a fluid that comes out from the volumetric measurement conduit when the aforementioned first fluid is filled in the volumetric measurement conduit,
   a third valve to be openably/closably connected to a third conduit that supplies a second fluid is further connected to a third part of the volumetric measurement conduit,
   a fourth valve to be openably/closably connected to a fourth conduit that communicates with a second container to which the first fluid in the volumetric measurement conduit and the aforementioned second fluid are sent is further connected to a fourth part of the volumetric measurement conduit,
   the third valve and the fourth valve each have a first port and a second port, an internal volume of the first port is smaller than an internal volume of the second port when the valves are closed,
   the first port of the third valve is connected to the aforementioned third part of the volumetric measurement conduit, and the first port of the fourth valve is connected to the aforementioned fourth part of the volumetric measurement conduit.
[3] The fluid volumetric measurement device of the aforementioned [2], wherein the third part is at the same position as the first part, adjacent to the first part, or at a position closer to the first part than the second part, and
   the fourth part is at the same position as the second part, adjacent to the second part, or at a position closer to the second part than the first part.
[4] The fluid volumetric measurement device of the aforementioned [2] or [3], further comprising a manifold, wherein
   the manifold has at least a valve connection face at which the first, second, third, and fourth valves are connected,
   a linear volumetric measurement conduit is provided in the inside of the manifold, and the volumetric measurement conduit has a first extension conduit linearly extending in the longitudinal direction from one edge part as the aforementioned third part, and a second extension conduit linearly extending in the longitudinal direction from the other edge part as the aforementioned fourth part,
   the first valve is configured on the valve connection face, and a conduit linearly connecting the first port of the first valve and the first part of the volumetric measurement conduit is provided in the manifold,
   the second valve is configured on the valve connection face, and a conduit linearly connecting the first port of the second valve and the second part of the volumetric measurement conduit is provided in the manifold,
   the third valve is configured on the valve connection face, and a conduit linearly connecting the first port of the third valve and an outside end part of the first extension conduit is provided in the manifold, and
   the fourth valve is configured on the valve connection face, and a conduit linearly connecting the first port of the fourth valve and an outside end part of the second extension conduit is provided in the manifold.
[5] The fluid volumetric measurement device of the aforementioned [4], wherein the volumetric measurement conduit is thicker than the first extension conduit or the second extension conduit.
[6] A sample analysis device comprising the fluid volumetric measurement device of any of the aforementioned [1] to [5], and comprising
   the first container containing the first fluid obtained by diluting a specimen liquid, and
   the second container containing a fluid obtained by diluting the aforementioned first fluid with the aforementioned second fluid,
   wherein the first container and the second container are connected by a conduit such that a predetermined amount of the first fluid in the first container is taken out and the predetermined amount of the first fluid is delivered to the second container together with a predetermined amount of the second fluid in the aforementioned sample analysis device.

### [Advantageous Effects of Invention]

In the present invention, a newly-found problem of volumetric measurement accuracy can be reduced by setting a specific direction as the direction of connection of ports of a valve. Consequently, a decrease in the volumetric measurement accuracy of fluid can be suppressed without using a special valve, as a result of which, for example, the accuracy of dilution is also improved.

The fluid volumetric measurement device of the present invention is constituted of a plurality of valves and conduits that connect the valves. Thus, by forming the conduits in a manifold rather than by piping using tubes, a more compact fluid volumetric measurement device not requiring labor of piping and a sample analysis device using the same are provided.

In the sample analysis device of the present invention, the fluid volumetric measurement device of the present invention is used for dilution (particularly, secondary dilution) of the first fluid (e.g., sample liquid). As a result, the accuracy of the volumetric measurement of the first fluid is further improved, which in turn also improves the accuracy of dilution. In addition, since the actions thereof are performed by opening and closing of the valve and movement of the liquid in the conduit, the dispensing action of the sampling nozzle is omitted from the secondary dilution step, and the operating time of the sample analysis device for each specimen is shortened.

### [Brief Description of Drawings]

Fig. 1 is a block diagram showing one example of the constitution of the fluid volumetric measurement device of the present invention, and one example of the constitution of the sample analysis device of the present invention using the fluid volumetric measurement device.
Fig. 2 is a sectional view schematically showing one example of the opening and closing structure of a general electromagnetic valve, for explanation of the internal volume of a port of a valve.

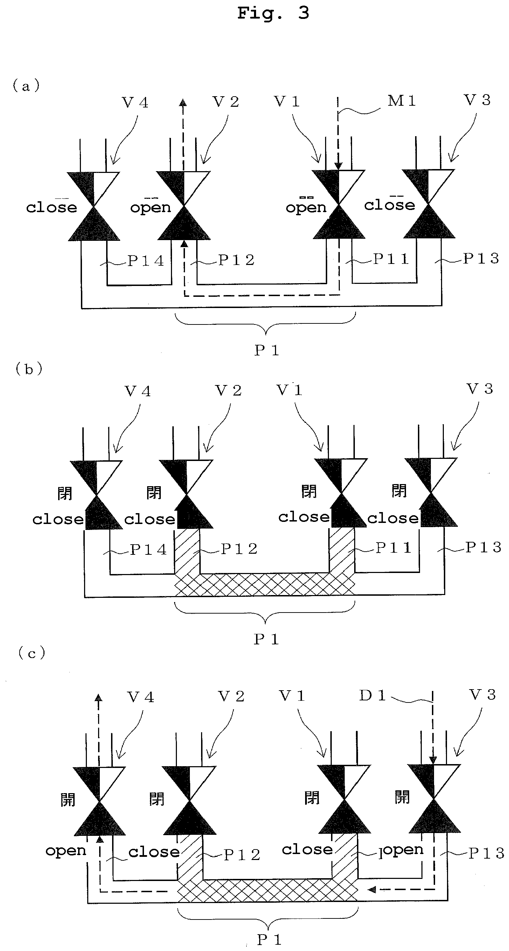

Fig. 3 is a schematic diagram showing how a sample liquid is diluted by the fluid volumetric measurement device of the present invention. In Fig. 3, for explanation, the valve is indicated by a symbol consisting of two triangles; of the two triangles for each valve, the triangle on the side of a port with a larger internal volume is indicated by a two-colored triangle consisting of a white half and a black half; and the triangle on the side of a port with a smaller internal volume is indicated by a black triangle. In addition, to earn easy understanding of the flow of liquid in the conduit, the conduit is drawn not as a line but as a band-like flow path with a certain width.
Fig. 4 is a flowchart showing procedures for diluting a sample liquid in the fluid volumetric measurement device of the present invention and the sample analysis device of the present invention.
Fig. 5 shows examples of various variations of the whole shape of the volumetric measurement conduit in the present invention.

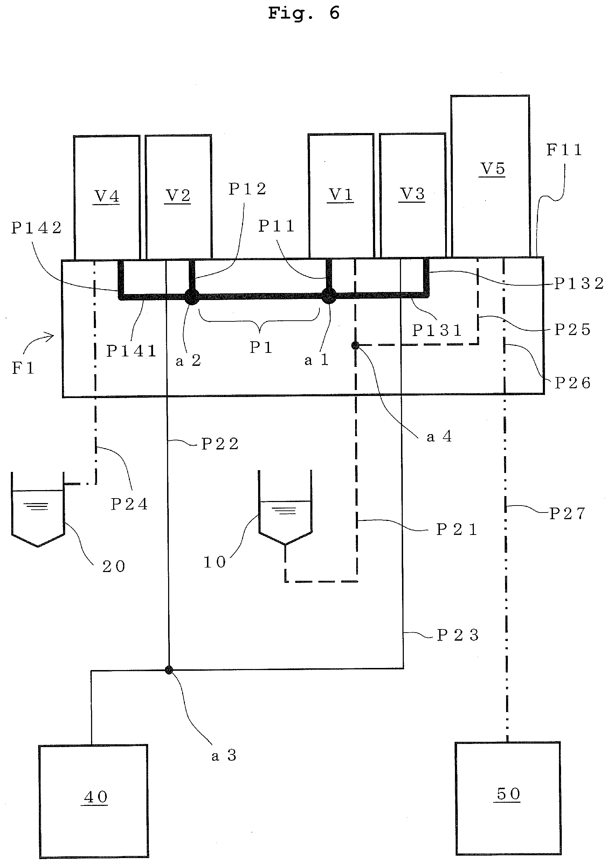

Fig. 6 is a block diagram showing an example in which the fluid volumetric measurement device of the present invention is constituted using a manifold. In the example of Fig. 6, a waste liquid recovering device including a suction device and a fifth valve for opening and closing a conduit connecting the first container and the waste liquid recovering device are further provided. In the drawing, thin straight lines, thick straight lines, broken lines, and dashed lines are used to ensure easy distinction of respective conduits, and even if these conduits intersect in the drawing, they are not actually connected to each other. The connection points between conduits are indicated by black circles.

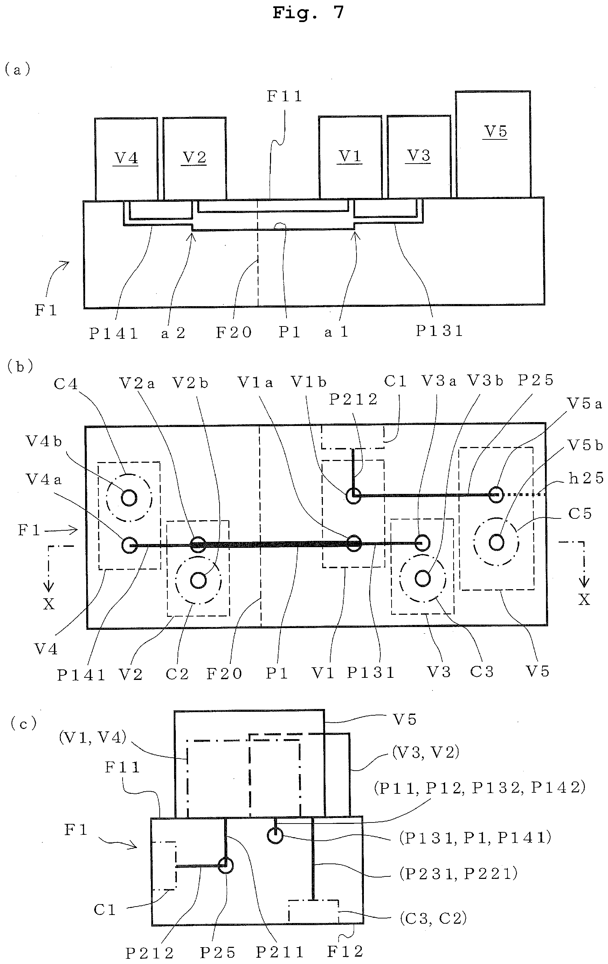

Fig. 7 shows a preferred embodiment of the manifold shown in Fig. 6. Fig. 7(a) is a sectional arrow view taken along the line X-X of Fig. 7(b) and shows a volumetric measurement conduit and extension conduits at both ends thereof. In Fig. 7(a), valve section is omitted and hatching showing the section is omitted. Fig. 7(b) shows the fluid volumetric measurement device of Fig. 7(a) viewed from below, and shows the positional relationship between the ports and conduits of each valve and the arrangement pattern of each valve. In Fig. 7(b), for explanation, each valve is indicated by a broken line, the port of each valve is indicated by a circle in solid line, the conduit in the manifold is indicated by a solid line, and the connectors provided on the side and bottom surfaces of the manifold are indicated by dashed lines. Fig. 7(c) is a side view of the fluid volumetric measurement device of Fig. 7(a) viewed from the right, and shows the route of the conduit in the manifold. In Fig. 7(c), the conduit in the manifold is indicated by a solid line, the conduit that extends orthogonally to the paper surface is indicated by a circle in solid line, the valve hidden behind the fifth valve is shown by a broken line and a dashed line, and the first and fourth valves and the second and third valves are shown at different heights for easy understanding.
Fig. 8 is a sectional view showing a preferred embodiment of the volumetric measurement conduit formed in the manifold shown in Fig. 6, Fig. 7. The hatching showing the section is omitted.

### [Description of Embodiments]

The constitution of the fluid volumetric measurement device of the present invention (to be also referred to as the fluid volumetric measurement device) is explained by referring to examples. In the following, the present invention is explained in an example using the first fluid as a sample liquid, the second fluid used as a fluid that extrudes the first fluid as a diluent, and the fluid volumetric measurement device for dilution. While the "fluid" is a liquid in the following example, the "fluid" in the present invention is not particularly limited and may also be, for example, a liquid containing a solid, a sol or the like.

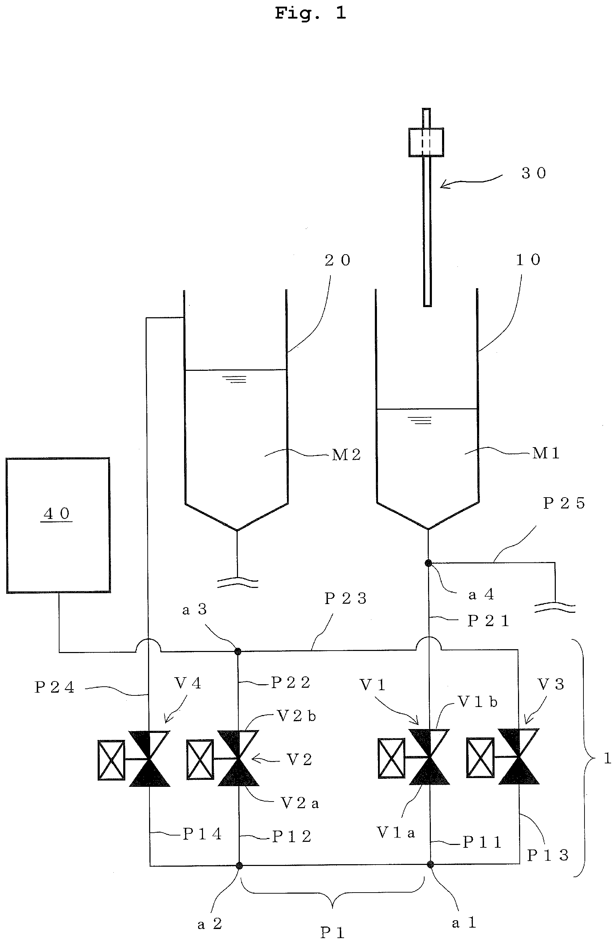

As shown in Fig. 1, the fluid volumetric measurement device 1 has at least a volumetric measurement conduit P1 with a predetermined internal volume, a first valve V1, and a second valve V2. The first valve V1 is connected to a first part a1 of the volumetric measurement conduit P1, and the second valve V2 is connected to a second part a2 of the volumetric measurement conduit P1. As used herein, the "part" in the volumetric measurement conduit refers to any position or region in the volumetric measurement conduit. In the example of Fig. 1, in one preferred embodiment, the volumetric measurement conduit P1 is a slender conduit, the first part a1 is located at one end (particularly, one edge part) of the volumetric measurement conduit P1, and the second part a2 is located at the other end (particularly, the other edge part) of the volumetric measurement conduit P1. The "end" and "the edge part" are described later.

Since the first valve V1 and the second valve V2 are connected to the first and second parts of the volumetric measurement conduit P1, when these valves are opened, a fluid from the outside (the first fluid described below) can flow through the volumetric measurement conduit P1, and when these valves are closed thereafter, the fluid that was in the volumetric measurement conduit is enclosed in the volumetric measurement conduit, whereby a predetermined volume of fluid is secured in the volumetric measurement conduit. These first and second valves open and close the conduits communicating with the first and second parts of the volumetric measurement conduit in response to a command from the control device (not shown). In addition, according to a command from the control device, a sample liquid is flown when the first and second valves are opened, and thereafter the sample liquid is enclosed in the volumetric measurement conduit by closing the first and second valves.

In the example of Fig. 1, the first valve V1 and the second valve V2 are opened, and a suction/supply device 40 including a suction source and a diluent (second fluid) supply source sucks a sample liquid M1 in a first container 10. The operation of each of these elements is controlled by a control device. As a result, the sample liquid M1 is filled in at least a conduit P21, the first valve V1, a conduit P11, and the volumetric measurement conduit P1. At this time, the sample liquid M1 is sucked until it reaches the inside of a conduit P12 and the second valve V2 so that the sample liquid M1 is filled in the measurement pipe P1 without any shortage. Then, the first valve V1 and the second valve V2 are closed. Depending on the accuracy of the suction device, the sample liquid may reach the inside of a conduit P22. To feed the sample liquid and then the diluent into the volumetric measurement conduit, further valves are connected to a third part and a fourth part of the volumetric measurement conduit (in the example of Fig. 1, both edge parts of the volumetric measurement conduit), an external suction source (pump, syringe, etc.), a diluent supply source, and the like are connected to the first and second valves and the aforementioned further valves, and they operate in response to a command from the control device. These are described later.

The first valve V1 has a first port V1a and a second port V1b, and the second valve V2 also has a first port V2a and a second port V2b. In these valves, the first port has a smaller internal volume than that of the second port when the valve is closed. The reason for the different internal volumes of the first port and the second port is described later.

In the present invention, it was first noted that the different internal volumes of the first port and the second port problematically causes a decrease in the volumetric measurement accuracy. To solve this problem, valves that close the volumetric measurement conduit at the first part (the first end, particularly, the first edge part in preferred embodiment) and the second part (the second end, particularly, the second edge part in preferred embodiment) are connected to the volumetric measurement conduit by a port with a smaller internal volume. That is, the first port V1a of the first valve V1 is connected to the first part a1 of the volumetric measurement conduit P1, and the first port V2a of the second valve V2 is connected to the second part a2 of the volumetric measurement conduit. As used herein, the (connection between the first port V1a of the first valve V1, and the first part a1 of volumetric measurement conduit P1), and the (connection between the first port V2a of the second valve V2, and the second part a2 of the volumetric measurement conduit P1) may be a direct connection, or a connection via a connection conduit with a predetermined microvolume. The predetermined microvolume may be a volume whose influence on dilution can be ignored, and is not particularly limited. For example, it is preferably a volume that does not affect the suppression of a decrease in the dilution accuracy, such as a volume equal to or less than the internal volume of the first port and the like, so that the purpose of the present invention can be achieved.

With the aforementioned connection, when a sample liquid filled in the volumetric measurement conduit is fed to an external conduit, the amount of the sample liquid flowing out from the ports V1a, V2a of the first and second valves becomes smaller than that when the ports V1b, V2b are connected to the volumetric measurement conduit. As a result, a decrease in the volumetric measurement accuracy is suppressed, and thus a decrease in the dilution accuracy is also suppressed.

### [volumetric measurement conduit]

In the present invention, the "volumetric measurement" refers to an operation to fill a space having a predetermined volume (space in the volumetric measurement conduit in the present invention) with a first fluid (e.g., sample liquid), thereby acquiring the first fluid with a predetermined volume.

The aforementioned predetermined volume can be determined in advance according to the volume and concentration of a diluted fluid of interest (a liquid obtained by further diluting the first fluid such as a sample liquid and the like). The volumetric measurement conduit is formed as one having a predetermined volume. The volume (predetermined volume) of the volumetric measurement conduit is not particularly limited. In a sample analysis device such as blood cell analysis device and the like, about 30 to 100 µL (µL represents microliter) is useful.

### [whole shape of volumetric measurement conduit]

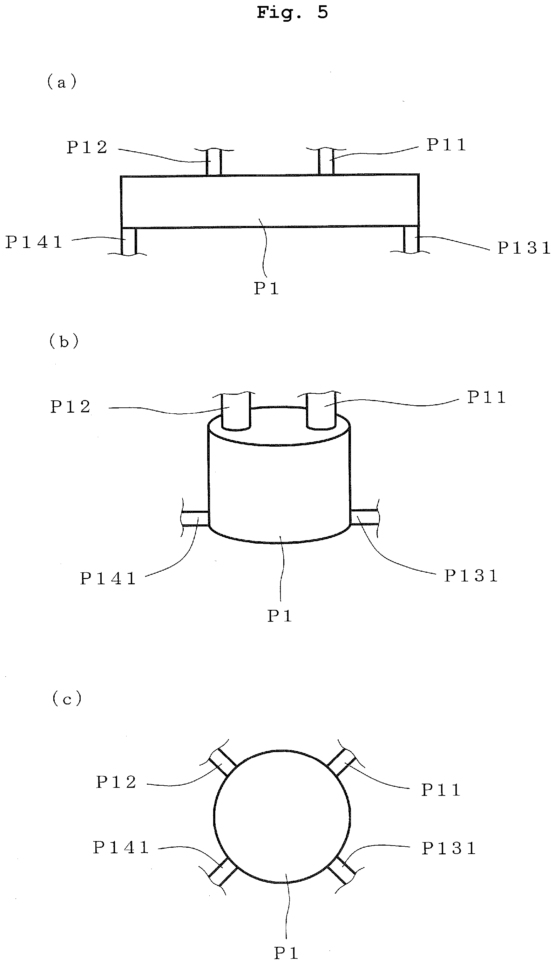

The whole shape (shape of whole internal space) of the volumetric measurement conduit is not particularly limited. An elongated shape like the volumetric measurement conduit P1 shown in Fig. 5(a) is preferable because calculation of the design volume is easy and the flow is less likely to stagnate when the first fluid is flown in or discharged. In particular, linear conduits are preferable because they are easy to process. Fig. 5(b), Fig. 5(c) show variations in the whole shape of the volumetric measurement conduit. In the example of Fig. 5(b), the whole shape of the volumetric measurement conduit is a columnar shape in which the central axis extends in the vertical direction of the Figure (cylindrical shape in the example of the Figure). In the example of Fig. 5(c), the whole shape of the volumetric measurement conduit is spherical. In addition, the whole shape of the volumetric measurement conduit may be an elliptical spherical shape, an elongated shape having a non-constant sectional shape, or the like.

When the whole shape of the volumetric measurement conduit is an elongated shape, the sectional shape thereof (shape of section when cut at right angles to the longitudinal direction of the conduit) may be a circular shape, a rectangle, or the like, and is not particularly limited. A circular shape is preferable from the aspects of processability and the influence on the flow of liquid. While the sectional shape is preferably constant, it may not be constant.

### [first to fourth parts of volumetric measurement conduit]

The first and the second parts of the volumetric measurement conduit are preferably in a position where the first fluid flows in smoothly and fills the volumetric measurement conduit smoothly, according to the whole shape of the volumetric measurement conduit. From this point, the whole shape of the volumetric measurement conduit is preferably an elongated shape, the first part is preferably one edge part of the volumetric measurement conduit, and the second part is the other edge part of the volumetric measurement conduit. The third and fourth parts of the volumetric measurement conduit are also preferably in a position where the first fluid filled in the volumetric measurement conduit can be extruded by the second fluid without retention. From this point, the third and fourth parts are also preferably the edge parts on the opposite sides of the elongated volumetric measurement conduit. These first to fourth parts can be appropriately determined according to the whole shape of the volumetric measurement conduit.

In a preferred embodiment, the third part may be at the same position as the first part, adjacent to the first part, or at a position closer to the first part than the second part. Similarly, the fourth part may be at the same position as the second part, adjacent to the second part, or at a position closer to the second part than the first part.

In the example of Fig. 5(a), the whole shape of the volumetric measurement conduit P1 is an elongated shape, and the first part (part connected to conduit P11) and the second part (part connected to conduit P12) of the volumetric measurement conduit are each located on the center side from the edge part of the volumetric measurement conduit P1. The third part (part connected to conduit P131) and the fourth part (part connected to conduit P141) are the both edge parts of the volumetric measurement conduit P1.

The end part of the volumetric measurement conduit as used in the present specification refers to, for example, a part closer to the edge than to the center in the longitudinal direction, and not only the edge but also any part in about 25% of the total length from the edge, when the whole shape of the volumetric measurement conduit is an elongated shape. In the end part of the volumetric measurement conduit, the part where the edge is present is referred to as the edge part. Since the conduit connected to the volumetric measurement conduit has a thickness, when the conduit is connected to the edge part of the volumetric measurement conduit, it means that the connection part includes the edge. Even when the whole shape of the volumetric measurement conduit is not an elongated shape and the longitudinal direction is absent, the end part and the edge may be defined on the wall surface of the volumetric measurement conduit with respect to any direction, as in the above.

In the example of Fig. 5(b), the whole shape of the volumetric measurement conduit P1 is a thick cylindrical shape with the center line facing up and down, in which the first part and the second part of the volumetric measurement conduit are located on the end face of the upper side of the cylinder, the third part and the fourth part are each located on the edge part on the lower side of the outer circumference of the cylindrical body, and the two are located at the opposing positions.

In the example of Fig. 5(c), the whole shape of the volumetric measurement conduit P1 is a sphere, in which the first part and the second part of the volumetric measurement conduit are located on the upper half side of the sphere, and the third part and the fourth part are located on the lower half side of the sphere.

In preferred embodiments shown in Fig. 6 - Fig. 8, the whole shape of the volumetric measurement conduit is an elongated linear shape, the first and second parts are each the both edge parts of the volumetric measurement conduit, the third part is the same edge part as the first part, and the fourth part is the same edge part as the second part. Such embodiment is preferable because the dead space becomes less, and the flow when the first fluid is filled in the volumetric measurement conduit and the flow of the second fluid (the flow extruding the first fluid from the volumetric measurement conduit) become smoother.

The volumetric measurement conduit may be a conduit (flow path in tube) constituted using a tube, or a conduit formed as a hole in a member, like the conduit in the below-mentioned manifold. By forming a volumetric measurement conduit in a manifold made of a hard material such as a metal material and the like, volume variation and production errors become smaller and the volumetric measurement accuracy becomes higher, as compared to a volumetric measurement conduit constituted of a soft tube.

### [valve]

In the present invention, the "valve" is a flow path opening and closing device that opens and closes a conduit, thus causing passage (open state) or shutting off (closed state) of a liquid in the conduit. It is a part (component, unit) having at least two ports (first port, second port), and a valve seat and a valve disc that allow internal communication between or shut off those two ports.

The valve used in the present invention is preferably one capable of performing an opening and closing action in response to a command from the control device. For example, an electromagnetic valve having a solenoid, motor or the like as an actuator is preferable. It may be a valve having an actuator that operates by a pneumatic pressure controlled by a control device, a valve having a piezo element (piezoelectric element) as an actuator (piezo valve) or the like. It is not limited to a valve that switches between full open and close in a binary manner, and a valve that can change the flow rate in the open state may be used. The control device for controlling the valve may be attached to the fluid volumetric measurement device. In a preferred embodiment, the control device of the sample analysis device in which the fluid volumetric measurement device is incorporated as a volumetric measurement device or a dilution device preferably controls the operation of the valve and controls the dilution of the sample liquid.

### [port of valve, and internal volume of port of valve]

The "port" of the valve in the present invention is a part of a hole provided in the valve and to which a flow path outside the valve is connected.

The "internal volume of the first port" of the valve in the present invention is a volume of a space from, when the valve is closed, the outside opening edge of the first port to the position of the valve disc (valve disc that allows communication between or shuts off space on the first port side and space on the second port side in the valve) (internal space of the first port). In other words, when the first port and the second port are shut off in the valve, it is the volume of a space on the valve inner side from the opening edge of the first port, where a fluid can stay continuously (internal space of the first port). Similarly, the "internal volume of the second port" is a volume of a space from, when the valve is closed, the outside opening edge of the second port to the valve disc (back side) (internal space of the second port). In other words, when the first port and the second port are shut off in the valve, it is the volume of a space on the valve inner side from the opening edge of the second port, where a fluid can stay continuously (internal space of the second port).

### [example of internal volume of port when valve is closed]

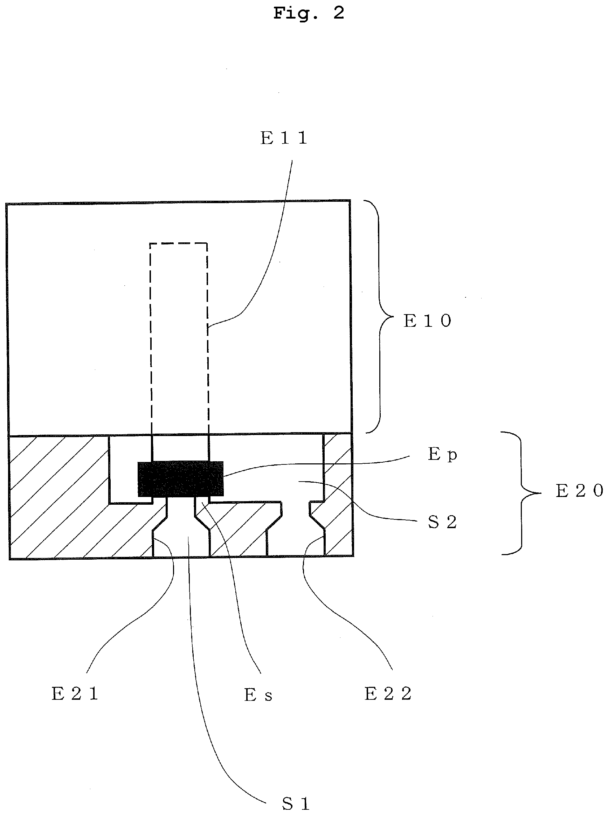

Fig. 2 is a sectional view schematically showing one example of the opening and closing structure of a valve (two-way valve). The Figure shows a two-way electromagnetic valve having a first port E21 with a small internal volume and a second port E22 with a large internal volume as a preferable example of the valve. The electromagnetic valve has an actuator part E10 and a valve structure part E20. In Fig. 2, the actuator part E10 is not in operation, a valve disc Ep closes the opening of a valve seat Es by a pressing force of a spring (not shown), whereby the first port E21 and the second port E22 are each shut off. When the actuator part E10 operates, a plunger E11 moves upward in the Figure against the spring force, the valve disc Ep fixed to the tip of the plunger E11 separates from the valve seat Es, and the two ports E21 and E22 communicate with each other.

As shown in Fig. 2, when the valve is closed, the volume of an internal space S1 of E21 (the first port), which is one of the two ports of the valve, is smaller than the volume of an internal space S2 of the other port (the second port) E22.

This is because the internal space S1 of the first port E21 is only the part of the inlet hole of the port (up to the lower surface of valve disc Ep), whereas the internal space S2 of the second port E22 includes not only the part of the inlet hole but also the internal space of the valve structure where the valve disc Ep exists. Since a male connector of the connection partner may be inserted in the part of the inlet hole of each port, the actual internal volume may be smaller by that amount.

As described above, in general two-way valves, even when the valve has a structure different from that of Fig. 2 (membrane valve, valve described in patent document 3, and the like), the internal volumes of the two ports are different from each other. The same applies to multi-port valves, as described later. In the example of Fig. 1, a general two-way electromagnetic valve is shown in which the internal volume of the first port (may be called "port A" or the like depending on the manufacturer and structure) is smaller than that of the second port (may be called "port P" or the like depending on the manufacturer and structure). In the example of Fig. 1, of the two triangles constituting the symbol of the two-way electromagnetic valve, the triangle on the first port side with a smaller internal volume is indicated by a black triangle, and the triangle on the second port side with a larger internal volume is indicated by a two-colored triangle consisting of a white half and a black half.

The internal volume of the first port is not always smaller than the internal volume of the second port, and depending on the valve structure, the internal volume of the first port is sometimes larger than the internal volume of the second port.

A valve obtained by integrally adding a connector to a port of a general commercially available valve may be interpreted as the port of the entire valve by adding the port of the connector to the port of the original valve. In addition, when the boundary between the original valve and the connector can be identified, the port after removal of the connector may be interpreted as the valve port.

When the second port E22, which is a port with a larger internal volume, is connected to the volumetric measurement conduit as shown in Fig. 2, a sample liquid enters the internal space S2 with a larger internal volume, the sample liquid flows out during dilution and mixes with a diluent, thus markedly lowering the concentration accuracy. It is known that the internal volumes of two ports in a valve may be different from each other. However, it was noted and found for the first time in the present invention that such difference in the internal volumes affects a decrease in the dilution accuracy. In the present invention, a port having a smaller internal volume is connected to the volumetric measurement conduit. As a result, when these valves are closed, the amount of the sample liquid in the port that may be added to the sample liquid in the volumetric measurement conduit is minimized, and the volumetric measurement accuracy of the sample liquid is improved.

The valve used in the present invention may be a valve having three or more ports. Two ports in such valve that are opened and closed can be utilized in the present invention. In such valve having three or more ports, the two ports used in the present invention have different internal volumes when the valve is closed, and a port having a smaller internal volume is connected to the first and second parts of the volumetric measurement conduit.

### [embodiment of preferable use of fluid volumetric measurement device]

Fig. 1 and Fig. 3 show a dilution device as a preferred embodiment of the fluid volumetric measurement device, in which the first and second valves, as well as the third and fourth valves are connected to the edge parts of the volumetric measurement conduit, so that a sample liquid will be introduced into the volumetric measurement conduit, then a diluent will be fed into the volumetric measurement conduit, and the sample liquid and the diluent will be charged from the volumetric measurement conduit. In the example of Fig. 1 and Fig. 3, the first valve V1 and the third valve V3 are connected to the first edge part a1 of the volumetric measurement conduit P1, and the second valve V2 and the fourth valve V4 are connected to the second edge part a2 of the volumetric measurement conduit P1. The third and fourth valves may be interchanged with each other.

A preferred embodiment is explained in the following with the first part of the volumetric measurement conduit P1 as the first edge part, and the second part as the second edge part.

The connection between valve and conduit in Fig. 1 is as follows.

The first valve V1 is a valve for openably/closably connecting the first edge part a1 of the volumetric measurement conduit P1 to a first conduit P21 communicating with a first container 10 containing a sample liquid M1. As the first container 10, for example, a leukocyte measurement chamber in a blood analysis device as a preferable example of the sample analysis device is preferable. In the example of Fig. 1, the first conduit P21 is connected to the bottom of the first container 10.

The second valve V2 is a valve for openably/closably connecting the second edge part a2 of the volumetric measurement conduit P1 to a second conduit P22. The second conduit P22 is a conduit to be an exit of a liquid that comes out from the volumetric measurement conduit when the sample liquid is filled in the volumetric measurement conduit. In the example of Fig. 1, the second conduit P22 is a conduit that sucks the sample liquid in the first container 10, and is connected to a suction/supply device 40 including a suction source. In an alternative embodiment, the sample liquid may be sent to the volumetric measurement conduit by the pressure from the first conduit P21.

The third valve V3 for openably/closably connecting to a third conduit P23 that supplies a diluent is further connected to one of the edge parts (first edge part a1 in Fig. 1), which is a third part of the volumetric measurement conduit P1. The third valve is connected to the first edge part a1 together with the first valve. That is, the first edge part a1 of the volumetric measurement conduit is also a connection point (merging/branching point) for the volumetric measurement conduit P1, a conduit P11, and a conduit P13. In the example of Fig. 1, the third conduit P23 is connected to the conduit P22 at a connection point a3 and connected to the suction/supply device 40.

The fourth valve V4 for openably/closably connecting to a fourth conduit P24 is further connected to the other edge part (second edge part a2 in Fig. 1) which is a fourth part of the volumetric measurement conduit P1. The fourth conduit P24 is connected to a second container 20, and is a conduit for sending the sample liquid M1 filled in the volumetric measurement conduit P1 and the diluent sent from the suction/supply device 40 to the second container 20.

The third valve V3 and the fourth valve V4 may be interchanged with each other. That is, the third valve V3 connected to the third conduit P23 that supplies the diluent may be connected to the second edge part a2 of the volumetric measurement conduit P1, and the fourth valve V4 connected to the second container 20 may be connected to the first edge part a1 of the volumetric measurement conduit P1.

The third valve V3 and the fourth valve V4 have, similar to the first and second valves, a first port and a second port, an internal volume of the first port is smaller than an internal volume of the second port when the valves are closed. Since the third and fourth valves are valves for flowing a diluent which is the second fluid, it is less important to direct their first ports toward the volumetric measurement conduit side as compared with the first and second valves. However, the sample liquid may also move toward the closed third and fourth valves. Thus, like the first and second valves, the first port of the third valve V3 is preferably connected to one of the edge parts which is the third part of the volumetric measurement conduit, and the first port of the fourth valve V4 of the volumetric measurement conduit is preferably connected to the aforementioned other edge part which is the fourth part of the volumetric measurement conduit. As is clear from the symbols indicating the first to fourth valves in Fig. 1, in the example of Fig. 1, in the first - fourth valves, the first port (black triangle) with a smaller internal volume is directed toward a volumetric measurement conduit P1, and connected to the volumetric measurement conduit. The connection in this case may also be a direct connection, or a connection via a connection conduit with a predetermined microvolume, like the connection of the first and second valves.

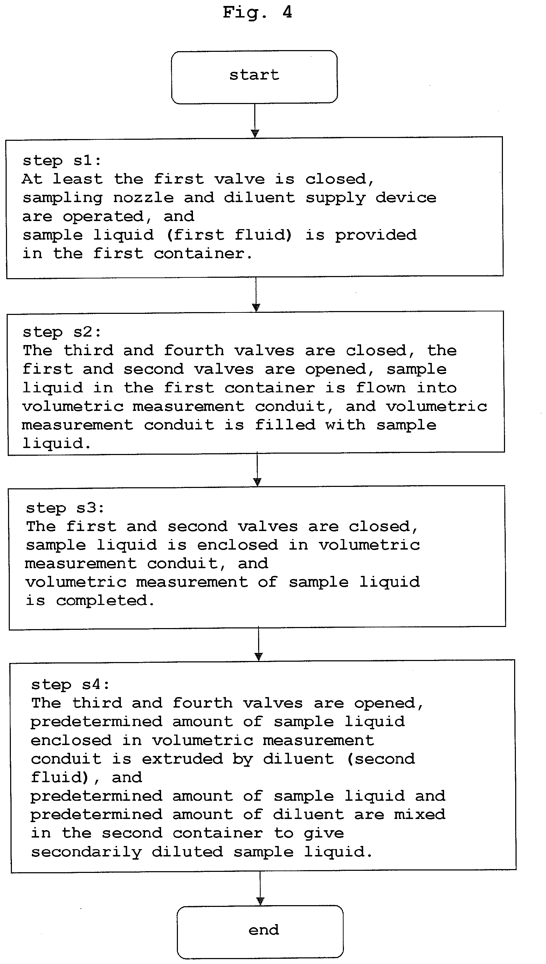

The volumetric measurement in Fig. 1 and Fig. 3, and successive dilution action are explained according to the flowchart shown in Fig. 4. In this example, the fluid volumetric measurement device is provided as a dilution device in a blood analysis device. In the example of Fig. 1, the sample liquid M1 is a blood specimen diluted with a diluent, the first container 10 is a WBC chamber, and the second container 20 is an RBC chamber. In the example of Fig. 1, the sample liquid M1 is produced in the first container. However, a sample liquid produced in other container may be transferred to the first container.

In a preferred embodiment, prior to the volumetric measurement of a sample liquid, the inside of each conduit is washed with a clean diluent. When the fluid volumetric measurement device is in operation and a sample liquid is flown in the volumetric measurement conduit, at least conduits P1, P11 - P14 are already filled with a clean diluent.

### [step s1: step of providing sample liquid M1]

First, in step s1, at least the first valve V1 is closed, a sampling nozzle 30 sucks a blood specimen from a specimen container (not shown), a predetermined amount of the blood specimen is discharged into the first container (WBC chamber) 10, a predetermined amount of a diluent is supplied from a diluent supplying device (not shown), and the mixture is stirred to provide the sample liquid M1 in the first container 10.

### [step s2: step of sending sample liquid M1 to volumetric measurement conduit]

Next, in step s2, as shown in Fig. 3(a), the third and fourth valves V3, V4 are closed, the first and second valves V1, V2 are opened, the suction/supply device 40 shown in Fig. 1 sucks the sample liquid M1 in the first container 10, and the volumetric measurement conduit P1 is filled with the sample liquid M1. To avoid insufficient filling of the sample liquid here, the sample liquid is sucked in excess, and therefore, the sample liquid M1 enters not only the volumetric measurement conduit but also the second valve V2. Since the conduits P13, P14 contain a diluent, the sample liquid M1 does not easily enter the conduits P13, P14. The diluent is contained in the conduits P13, P14 because when the first liquid is placed in the volumetric measurement conduit with the air present in the conduits P13, P14, the first liquid also enters the conduits P13, P14 due to the large compression deformation of the air, which in turn increases error in the volumetric measurement.

In contrast, when the conduits P13, P14 contain a diluent, the sample liquid M1 does not easily enter the conduits P13, P14. Thus, in a preferred embodiment, a diluent is placed in advance in the conduit P1, conduits P13, P14 before the aforementioned step s1.

### [step s3: step of completing volumetric measurement of sample liquid]

Next, in step s3, as shown in Fig. 3(b), the first and second valves V1, V2 are closed, and the sample liquid M1 is enclosed in the volumetric measurement conduit P1, whereby volumetric measurement of the sample liquid is completed. At this time, the first port V1a of the first valve V1 and the conduit P11 are also filled with the sample liquid M1, and the conduit P12 and the first port V2a of the second valve V2 are also filled with the sample liquid M1.

### [step s4: diluting step]

Next, in step s4, as shown in Fig. 3(c), the third and fourth valves V3, V4 are opened, a predetermined amount of the sample liquid M1 enclosed in the volumetric measurement conduit P1 is extruded by a diluent D1 sent in a predetermined amount from the suction/supply device 40 shown in Fig. 1, the predetermined amount of the sample liquid M1 and the predetermined amount of the diluent D1 pass through a conduit P14, the fourth valve V4, and the conduit P24, are sent into the second container 20, where they are mixed to give a diluted sample liquid M2 to complete the dilution. When the sample liquid in the volumetric measurement conduit is extruded by the diluent, an indefinite amount of the sample liquid M1 flows out from the first port V1a of the first valve V1, the conduit P11, the first port V2a of the second valve V2, and the conduit P12, and sent into the second container 20. However, since the first port V1a of the first valve V1 and the first port V2a of the second valve V2 are ports with a smaller internal volume, the amount of the sample liquid M1 flow out is small and a decrease in the volumetric measurement, and further, dilution accuracy is suppressed.

In various sample analysis devices such as blood analysis device and the like, many specimen liquids are diluted and measured one after another by washing measurement chambers and pipings in the device. In the explanation of the aforementioned step s2, it is stated, "when the conduits P13, P14 contain a diluent, the sample liquid M1 does not easily enter the conduits P13, P14. Thus, in a preferred embodiment, a diluent is placed in advance in the conduit P1, conduits P13, P14 before the aforementioned step s1". In a sample analysis device, when a certain one sample liquid M1a is diluted, the third and fourth valves V3, V4 are opened and a diluent extrudes the sample liquid M1a in the volumetric measurement conduit P1 in the aforementioned step s4. At this time, the diluent remains in the conduits P13, P14 in Fig. 3(c). The diluent remaining in the conduits P13, P14 may be used as (diluent to be placed in the conduits P13, P14 in advance when a sample liquid M1b to be measured after the sample liquid M1a is diluted). Alternatively, when the measurement relating to the sample liquid M1a is completed, valve V1 - conduit P11 - volumetric measurement conduit P1 - conduit P12 - valve V2 are washed with a diluent for the next sample liquid M1b. Simultaneously with the washing, or after the washing, the conduits P13, P14 may be separately washed, and the diluent remaining in the conduits P13, P14 may be used as (diluent to be placed in the conduits P13, P14 in advance when the sample liquid M1b to be measured after the sample liquid M1a is diluted).

### [constitution of fluid volumetric measurement device using manifold]

As shown in Fig. 6 - Fig. 8, in a preferred embodiment, the fluid volumetric measurement device has a manifold, and each conduit is formed in the manifold.

In the present invention, the "manifold" is a member provided with a valve connection port corresponding to each port of plural valves so that, the plural valves can be attached and connected. The manifold has a body composed of structural materials such as resin material, metal material and the like, is provided with a conduit necessary for the fluid volumetric measurement device as an internal conduit in the inside of the body and, where necessary, an external port for connection with an external conduit on the external surface.

By attaching and connecting plural valves to the manifold, ports of a plurality of predetermined valves can be connected to each other through the internal conduit without the need for piping, as well as the port of a predetermined valve can be easily connected to an external conduit through an external port. When plural manifolds are used to connect valves, each port of the valve is also constituted such that it is connected to a valve connection port by simple mounting on the valve connection face of the manifold.

Assembling plural valves into one block using a manifold is known in itself. However, the manifold used in the present invention is constituted exclusively for the fluid volumetric measurement device which has not existed before. The manifold has a volumetric measurement conduit inside, and has a conduit to connect the volumetric measurement conduit and the port of each valve. As a result, the advantages of the fluid volumetric measurement device can be shown further remarkably, in addition to the advantages of conventional manifolds.

As shown in Fig. 6, Fig. 7, a manifold F1 used in the fluid volumetric measurement device has a valve connection face F11 to which at least the first, second, third, and fourth valves (V1 - V4) can be connected. In the example of Fig. 6, Fig. 7, the fifth valve V5 is further connected, and conduits P25, P26 for the fifth valve V5 are provided in the manifold. In the inside of the manifold, a linear volumetric measurement conduit P1 is provided in parallel to the valve connection face F11. The "linear" here is not limited to a strict straight line, and may be a substantially straight line within a range according to the processing method and processing accuracy. The "parallel" is not limited to being strictly parallel, and includes a state in which the volumetric measurement conduit P1 has a substantially constant distance from the valve connection face F11 over the entire longitudinal direction. The volumetric measurement conduit P1 has a first extension conduit P131 linearly extending from one edge part a1 in the longitudinal direction of the volumetric measurement conduit P1, and a second extension conduit P141 linearly extending from the other edge part a2 in the longitudinal direction of the volumetric measurement conduit P1. As a result, the first extension conduit P131, the volumetric measurement conduit P1, and the second extension conduit P141 extend in a straight line.

Since the volumetric measurement conduit P1 is linear, the flow becomes smooth, and further, since the first extension conduit P131, the volumetric measurement conduit P1, and the second extension conduit P141 are in a straight line, the flow becomes smoother.

### [relationship between valve and internal conduit]

The first valve V1 is configured on the valve connection face F11 such that the first port is positioned directly above the first edge part a1 of the volumetric measurement conduit P1. The manifold F1 is provided with a conduit P11 that linearly connects the first port of the first valve V1 and the first edge part a1 of the volumetric measurement conduit P1. When the first valve V1 is configured at a predetermined position of the valve connection face F11, the first port of the first valve V1 and a valve connection port (detail shape is not shown) formed in the opening of the conduit P11 are joined. For the seal structure of the joining part, a conventionally-known connection structure between a manifold and a valve may be referred to. The same applies to the connection structure between other valves and the manifold F1.

The second valve V2 is configured on the valve connection face F11 such that the first port is positioned directly above the second edge part a2 of the volumetric measurement conduit P1. The manifold F1 is provided with a conduit P12 that linearly connects the first port of the second valve V2 and the second edge part a2 of the volumetric measurement conduit P1.

The third valve V3 is configured on the valve connection face F11 such that the first port is positioned directly above an outside edge part (edge part farther from volumetric measurement conduit) of the first extension conduit P131. The manifold is provided with a conduit P132 that linearly connects the first port of the third valve V3 and the outside edge part of the first extension conduit P131, and the extension conduit P131 and the conduit P132 are connected to form the conduit P13 shown in Fig. 1 and Fig. 3.

The fourth valve V4 is configured on the valve connection face F11 such that the first port is positioned directly above an outside edge part of a second extension conduit P141. The manifold is provided with a conduit P142 that linearly connects the first port of the fourth valve V4 and the outside edge part of the second extension conduit P141, and the extension conduit P141 and the conduit P142 are connected to form the conduit P14 shown in Fig. 1 and Fig. 3.

As shown in Fig. 6, internal conduits to be respectively connected to external conduits P21, P22, P23, P24, P27 are provided in the manifold F1, and these internal conduits are provided with external ports on the external surface of the manifold. The illustration of the structure of the external port is omitted.

### [material of manifold]

The material of the body of the manifold F1 is not particularly limited and may be the same as the material of conventionally-known manifolds. Resin materials such as PET, nylon resin, acrylic resin and the like are preferable from the aspects of corrosion resistance to sample liquid, processability of holes and grooves, material cost, mechanical strength, surface condition (roughness), and the like.

### [method for forming manifold]

As a method for forming the internal conduit of a manifold, hole forming from the external surface is simple and preferable. Where necessary, the manifold is divided into plural blocks, hole forming is performed from the divided surface (surface to be joined), the plurality of blocks are combined, and the groove may be used as the internal conduit. In addition, hole forming is performed from the external surface of the manifold, and the part that opens to the external surface is sealed by brazing, adhesive, plug, or the like to embed the conduit in the manifold. Alternatively, the manifold is divided into plural blocks, a groove to be the conduit is formed on the joined surface, and plural blocks are joined by bolting, welding, brazing, adhesive, thermocompression bonding, or the like to embed the groove as an internal conduit.

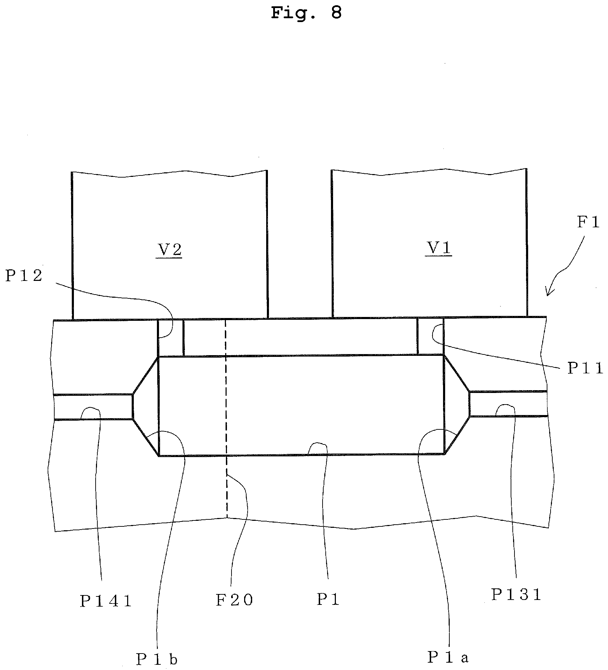

In the example of Fig. 7, Fig. 8, the body of the manifold is divided into two by a divided face F20 shown with a broken line, and a volumetric measurement conduit P1, a first extension conduit P131, and a second extension conduit P141 are formed by hole forming from the divided face F20. As shown in Fig. 7(b), an internal conduit P25 is formed by hole forming from the external surface of the manifold, a part h25 that opens to the external surface is closed.

In addition, it is not limited to cutting, and a member having the same shape as the above-mentioned manifold may be formed by molding.

### [embodiment of preferable configuration of valve in manifold]

Fig. 7 schematically shows preferable configuration of valves in a manifold F1. In the example of Fig. 7, four valves V1 - V4 are arranged next to one another in one direction. In addition, in the manifold F1, internal conduits are formed according to the configuration of the valves so that the configuration is possible. As clearly shown in Fig. 7(b), in this embodiment, respective valves (V1 - V5) are configured on a valve connection face F11 so that the ports (V1a, V2a, V3a, V4a, V5b) on one side are arranged in one line. As mentioned above, the first ports (V1a, V2a, V3a, V4a) of the valves (V1 - V4) are located directly above a volumetric measurement conduit and extension conduits thereof. Other ports (V1b, V2b, V3b, V4b, V5a) of each valve are alternately located on the opposite side of the straight line connecting the ports on one side, and are located in a zigzag pattern. That is, the positional relationship between the two ports of the adjacent valves is opposite to each other between the valves. By such configuration of the valves, first, the volumetric measurement conduit and the first and second extension conduits on both sides thereof can be arranged in one line, and the first ports of the first - fourth valves (V1 - V4) can be connected to the volumetric measurement conduit and extension conduit directly below by the shortest linear conduit. Furthermore, since the second ports of each adjacent valve are separated from each other with a sufficient distance due to the zigzag configuration, in a surface F12 on the opposite side from the valve connection face F11, external ports (C2, C3, C4, C5) with large holes can be arranged at positions corresponding to directly below each of the second ports without interfering with each other. A conduit P211 (Fig. 7(c)) extending directly below the second port V1b of the first valve V1 joins a conduit P25 extending from the first port V5a of the fifth valve V5 to form a conduit P212 which is connected to an external port C1 at side. The external port C1 is connected to the first container 10.

With the above-mentioned constitution, a compact fluid volumetric measurement device (dilution device) having a necessary external port while shortening the unnecessary internal conduits other than the volumetric measurement conduit can be obtained.

Fig. 7(c) is a side view supplementing the positional relationship between the internal conduit and the external port shown in Fig. 7(b). In this drawing, the first and fourth valves appear to overlap, and the second and third valves appear to overlap. The conduits (P11, P12, P132, P142) directly below which are connected to the first port of each of the first - fourth valves appear to overlap. The conduits (P231, P221) directly below which are connected to the second port of each of the third and second valves (V3, V2) appear to overlap. The volumetric measurement conduit P1, the first extension conduit P131, and the second extension conduit P141 appear to overlap at one point.

### [preferred embodiment of volumetric measurement conduit]

As shown in Fig. 8, in a preferred embodiment of the fluid volumetric measurement device, a volumetric measurement conduit P1 is thicker than the first and second extension conduits, and therefore, the full-length is shorter by that amount. Since the volumetric measurement conduit is thick and short even though it has a predetermined volume, the area of the internal surface of the volumetric measurement conduit becomes smaller. As a result, frictional resistance of liquids becomes small and the adsorption of components contained in a sample liquid to the internal surface of the volumetric measurement conduit can be reduced. In addition, since the first and second extension conduits are thinner than the volumetric measurement conduit, the sample liquid does not easily enter the first and second extension conduits P131, P141 when it flows from a conduit P11 to a conduit P12 through the volumetric measurement conduit P1. As a result, a decrease in the measurement accuracy is further suppressed.

In the embodiment of Fig. 8, the volumetric measurement conduit P1, and the first and second extension conduits P131, P141 are preferably connected by taper parts P1a, P1b in consideration of smooth flow of the liquid. The taper parts P1a, P1b preferably have a shape connecting the entire circumferential direction of the edge part of the internal wall surface of the volumetric measurement conduit P1, and the entire circumferential direction of the edge part of the internal wall surface of each extension conduit, and the volumetric measurement conduit P1, and at least one of the first and second extension conduits P131, P141 are more preferably almost coaxial. The taper parts P1a, P1b may be interpreted as belonging to any of the volumetric measurement conduit and the extension conduit, and can be determined in consideration of an influence on the volumetric measurement. In the example of Fig. 8, the taper parts P1a, P1b belong to an extension conduit, and a straight conduit part of the volumetric measurement conduit has a predetermined volume.

### [sample analysis device of the present invention]

Next, the constitution of the sample analysis device of the present invention (also referred to as the sample analysis device) is explained by way of an example.

As shown in Fig. 1, Fig. 6, the sample analysis device is constituted using the above-mentioned fluid volumetric measurement device of the present invention. In the example of Fig. 6, a manifold is used for the fluid volumetric measurement device. The sample analysis device has the first container 10 and the second container 20. The first container 10 is a container for accommodating a sample liquid M1 obtained by diluting a specimen liquid. In the example of Fig. 1, it is a WBC chamber. The second container 20 is a container for accommodating a sample liquid M2 obtained by further diluting (secondary dilution) the sample liquid M1. In the example of Fig. 1, it is an RBC chamber. The fluid volumetric measurement device 1 of the present invention is connected to the first container 10 and the second container 20 via conduits P21, P24, so that a predetermined amount of the sample liquid M1 in the first container 10 will be taken out, and the predetermined amount of the sample liquid M1 will be sent to the second container 20 together with a predetermined amount of a diluent.

The sample analysis device further has one or more measurement chambers that perform various measurements for sample analysis and a dispensing mechanism that provides a sample liquid to the measurement chambers, and also has a control device for operating the measurement chamber, the dispensing mechanism, and the fluid volumetric measurement device of the present invention, and processing and analyzing the measurement result signals. In the example of Fig. 1, only two measurement chambers (WBC chamber, RBC chamber) are shown for explanation, and these are the first container and the second container for the fluid volumetric measurement device. The WBC chamber is a measurement chamber constituted to perform measurements such as counting and the like for the analysis of leukocytes, and the RBC chamber is a measurement chamber constituted to perform measurements such as counting and the like for the analysis of erythrocytes and the like. These chambers are provided with a device for the measurement of particles based on an electrical resistance method and flow cytometry, and the measurement results signals are sent to the control device.

### [dispensing mechanism]

Fig. 1 shows a sampling nozzle 30 as a part of the dispensing mechanism. The sampling nozzle 30 is an elongated tube that moves to a specimen container (not shown), a reagent container provided as needed, or other measurement chamber, and performs suction and discharge of sample liquid, reagent liquid and the like under the control of the control device. The sampling nozzle is attached to a transfer mechanism so that it can move in the horizontal direction and the vertical direction. The upper end of the sampling nozzle is connected to the suction-discharge pump through a conduit, an electromagnetic valve or the like. For these transfer mechanism, piping, mechanism for supplying a diluent, a container for accommodating a specimen liquid, a container for accommodating necessary reagents, and the like, the prior art can be referred to and illustrations are omitted.

### [sample liquid and analysis]

In the example of Fig. 1, a sample liquid M1 is a liquid obtained by primarily diluting a specimen liquid with a diluent. The specimen is not particularly limited, and includes general body fluids such as blood, urine, sweat, saliva and the like.

The analysis items by the sample analysis device are not particularly limited. In the case of blood analysis device, for example, haematological analyses such as leukocyte count (including classification), erythrocyte count, hemoglobin concentration and the like, biochemical analyses such as C-reactive protein (CRP) concentration and the like, immunological analysis, genetic analysis and the like can be mentioned. For these analyses, necessary measurement chambers such as CRP measurement chamber and the like may be provided as appropriate.

As mentioned above, in blood analysis device, blood specimens are often secondarily diluted, and the dilution requires high accuracy of volumetric measurement. Therefore, the usefulness of the fluid volumetric measurement device of the present invention becomes more remarkable when it is used as a dilution device in a blood analysis device.

### [diluent as the second fluid]

When the second fluid is a diluent, as the diluent, those used in conventionally-known sample analysis devices can be referred to. For example, those containing water, sodium chloride, sodium fluoride, sodium azide and the like as components can be mentioned.

The second fluid is not limited to the aforementioned diluents alone, and may be a fluid that extrudes the first fluid, and gasses such as air and the like may be used. However, since air is easily compressed and deformed, and causes water droplets on the inner wall of conduits, a liquid that is not easily compressed or deformed is more preferable.

### [dilution rate]

When the fluid volumetric measurement device is used as a dilution device, the dilution rate by the dilution device also varies depending on the analysis item. In the case of, for example, blood analysis device, a blood specimen is diluted about 150-fold to 200-fold by the first container WBC chamber. The diluted blood specimen (sample liquid) is diluted about 50-fold to 100-fold by the fluid volumetric measurement device. The control device operates the pump of each part to achieve the dilution, and controls the amount of a blood specimen to be supplied to the first container, the amount of a diluent to be supplied, and the amount of a diluent to be supplied to the fluid volumetric measurement device.

### [other valve]

In the example of Fig. 1, Fig. 6, a conduit P25 is connected to the lower part of the first container, and the liquid in the first container 10 is discharged through the conduit P25, and air is fed to stir the liquid in the first container. As shown in Fig. 6, Fig. 7, therefore, the sample analysis device is provided with the fifth valve and connected to a waste liquid recovering device 50 via conduits P25, P26, P27. The waste liquid recovering device 50 includes a pump, sucks the liquid in the first container 10, and operates to discharge same into the waste liquid container. The waste liquid recovering device 50 can also supply air to stir the liquid in the first container by switching the valve.

### [control device]

The control device may be constructed with a logic circuit and the like, but a computer is suitable. In the computer, the actions of each part of the fluid volumetric measurement device and particle analysis device of the present invention are controlled, and a program configured to perform arithmetic operations for analysis is executed.

A power source, a pneumatic pressure source, a driver, an external measurement device, a motor, and the like for operating each part in response to a command (electric signal) from the control device may be provided as appropriate.

When the sample analysis device is a blood analysis device, conventionally-known analysis devices of, for example, patent document 1, JP 2014-224754 A ("apparatus for measuring blood cells and immunity from whole blood") and the like can be referred to for the mechanism of details such as specimen liquid dispensing mechanism, diluent supply mechanism, reagent supply mechanism and the like, constitution of various measurement chambers, and the contents of the operation under a command from the control device.

### [Industrial Applicability]

According to the present invention, a fluid volumetric measurement device that suppresses a decrease in the volumetric measurement accuracy of fluid is provided, and a sample analysis device provided with the fluid volumetric measurement device is provided. Therefore, the sample analysis device achieves a low price by using an inexpensive general valve, suppresses a decrease in the volumetric measurement accuracy, also suppresses a decrease in the dilution accuracy, and shortens the operating time for dilution, and is preferable.

This application is based on a patent application No. 2018-246184 filed in Japan (filing date: December 27, 2018), the contents of which are incorporated in full herein.

### [Explanation of Symbols]

- 1: fluid volumetric measurement device
- 10: the first container
- 20: the second container
- 30: sampling nozzle
- 40: suction/supply device
- M1: sample liquid
- M2: diluted sample liquid
- V1: the first valve
- V2: the second valve
- V3: the third valve
- V4: the fourth valve

## Claims

1. A fluid volumetric measurement device comprising at least a volumetric measurement conduit with a predetermined volume, a first valve connected to a first part of the volumetric measurement conduit, and a second valve connected to a second part of the volumetric measurement conduit, wherein
the first valve and the second valve each have a first port and a second port, an internal volume of the first port is smaller than an internal volume of the second port when the valves are closed,
the first port of the first valve is connected to the first part of the volumetric measurement conduit, and the first port of the second valve is connected to the second part of the volumetric measurement conduit.

2. The fluid volumetric measurement device according to claim 1, wherein the first valve is a valve for openably/closably connecting the first part of the volumetric measurement conduit to a first conduit communicating with a first container containing a first fluid,
the second valve is a valve for openably/closably connecting the second part of the volumetric measurement conduit to a second conduit to be an exit of a fluid that comes out from the volumetric measurement conduit when the first fluid is filled in the volumetric measurement conduit,
a third valve to be openably/closably connected to a third conduit that supplies a second fluid is further connected to a third part of the volumetric measurement conduit,
a fourth valve to be openably/closably connected to a fourth conduit that communicates with a second container to which the first fluid in the volumetric measurement conduit and the second fluid are sent is further connected to a fourth part of the volumetric measurement conduit,
the third valve and the fourth valve each have a first port and a second port, an internal volume of the first port is smaller than an internal volume of the second port when the valves are closed,
the first port of the third valve is connected to the third part of the volumetric measurement conduit, and the first port of the fourth valve is connected to the fourth part of the volumetric measurement conduit.

3. The fluid volumetric measurement device according to claim 2, wherein the third part is at the same position as the first part, adjacent to the first part, or at a position closer to the first part than the second part, and
the fourth part is at the same position as the second part, adjacent to the second part, or at a position closer to the second part than the first part.

4. The fluid volumetric measurement device according to claim 2 or 3, further comprising a manifold, wherein
the manifold has at least a valve connection face at which the first, second, third, and fourth valves are connected,
a linear volumetric measurement conduit is provided in the inside of the manifold, and the volumetric measurement conduit has a first extension conduit linearly extending in the longitudinal direction from one edge part as the third part, and a second extension conduit linearly extending in the longitudinal direction from the other edge part as the fourth part,
the first valve is configured on the valve connection face, and a conduit linearly connecting the first port of the first valve and the first part of the volumetric measurement conduit is provided in the manifold,
the second valve is configured on the valve connection face, and a conduit linearly connecting the first port of the second valve and the second part of the volumetric measurement conduit is provided in the manifold,
the third valve is configured on the valve connection face, and a conduit linearly connecting the first port of the third valve and an outside end part of the first extension conduit is provided in the manifold, and
the fourth valve is configured on the valve connection face, and a conduit linearly connecting the first port of the fourth valve and an outside end part of the second extension conduit is provided in the manifold.

5. The fluid volumetric measurement device according to claim 4, wherein the volumetric measurement conduit is thicker than the first extension conduit or the second extension conduit.

6. A sample analysis device comprising the fluid volumetric measurement device according to any one of claims 1 to 5, and comprising
the first container containing the first fluid obtained by diluting a specimen liquid, and
the second container containing a fluid obtained by diluting the first fluid with the second fluid,
wherein the first container and the second container are connected by a conduit such that a predetermined amount of the first fluid in the first container is taken out and the predetermined amount of the first fluid is delivered to the second container together with a predetermined amount of the second fluid in the sample analysis device.
